## Europäisches Patentamt
## European Patent Office
## Office européen des brevets

(19)

(11) Veröffentlichungsnummer: **0 001 285**
**B1**

(12)
# EUROPÄISCHE PATENTSCHRIFT

(45) Veröffentlichungstag der Patentschrift: **09.06.82**

(21) Anmeldenummer: **78100950.1**

(22) Anmeldetag: **21.09.78**

(51) Int. Cl.³: **A 61 L 2/02,** A 61 L 2/24, C 02 F 1/46

(54) **Vorrichtung zur Entkeimung von Flüssigkeiten.**

(30) Priorität: **21.09.77 DE 2742508
23.12.77 DE 2757854**

(43) Veröffentlichungstag der Anmeldung:
**04.04.79 Patentblatt 79/7**

(45) Bekanntmachung des Hinweises auf die Patenterteilung:
**09.06.82 Patentblatt 82/23**

(84) Benannte Vertragsstaaten:
**BE CH DE FR GB LU NL SE**

(56) Entgegenhaltungen:
**CH - A - 319 956
CH - 560 170
DE - A - 2 311 504
FR - A - 2 224 207
FR - A - 2 283 699**

(73) Patentinhaber: **Reis, August K., Prof. Dr. med.
Faistenbergerstrasse 1
D-8000 München 90 (DE)**

(72) Erfinder: **Reis, August K., Prof. Dr. med.
Faistenbergerstrasse 1
D-8000 München 90 (DE)**
Erfinder: **Kirmaier, Norbert L., Dr.-Ing.
Mondstrasse 6
D-8011 Aschheim (DE)**
Erfinder: **Schöberl, Meinolf, Dipl.-Ing.
Geigelstein strasse 8
D-8210 Prien/Chiemsee (DE)**

Courier Press, Leamington Spa, England.

Vorrichtung zur Entkeimung von Flüssigkeiten

Die Erfindung bezieht sich auf eine Vorrichtung zur Entkeimung von Flüssigkeiten mittels der anodischen Oxidation mit einem Flüssigkeitseingang im unteren Innenraumbereich und einem Ausgang im oberen Innenraumbereich und einer Mehrzahl von durchströmten bzw. umströmten Haupt- und Hilfselektroden, wobei die Hauptelektroden am stromabwärtsgelegenen Eingang und am stromaufwärtsgelegenen Ausgang des Elektrodenbereiches sich über den ganzen Strömungsquerschnitt erstrecken und Unterbrechungen zum Durchtritt der Flüssigkeit aufweisen und mit einem an den Hauptelektroden anliegenden in seiner Richtung periodisch umschaltbaren Potential.

Eine Vorrichtung gemäß des Oberbegriffes ist beispielsweise aus der DE—OS 23 37 355 bekannt. Zwischen den Hauptelektroden ist eine Anzahl von mittels von in dem Strömungsbereich vorgesehenen Kunststoffteilen gehaltener kugelförmiger Hilfselektroden angeordnet. Bei dieser Anordnung ist eine einwandfreie Verwirbelung und Abscheidung der sich bildenden Gasblasen nich gewährleistet. Zum Reinigen der Elektroden ist ihre Polarität in zeitlichen Abständen umkehrbar. Gewünschtenfalls kann die Elektrolyse auch mit Wechselstrom durchgeführt werden. Dabei wird in Kauf genommen, daß die dann einsetzende Umpolarisation mit einer gewissen Trägheit erfolgt und so die Entkeimung bei jedem Umschalten unterbrochen wird. Diese Vorrichtung kann daher nicht zur anodischen Oxidation zur Entkeimung von Wasser für die Pharmazie oder medizinischen Bereiche verwendet werden, weil eine solche Unterbrechung nicht möglich ist.

Aus der US—PS 33 35 078 ist eine bipolare Zelle bekannt, die als oben offene Laufrinne ausgebildet ist und bei der in gewissen Abständen aufeinanderfolgend senkrecht übereinander angeordnete Elektroden miteinander und mit der Spannungsquelle leitend verbunden sind. Die Elektrodenkontaktanschlüsse sind im Strömungsbereich als konstruktives Element vorgesehen. Durch die dadurch entstehende Feldimhomogenität wird der Wirkungsgrad dieses Oxidationsreaktors vermindert. Sowohl durch die Anordnung der Kontaktanschlüsse im Strömungsbereich als auch durch die waagerechte Strömungsrichtung, bei der die Blasen vom Boden zur oberen Schicht der Hilfselektroden fließen, wird eine inhomogene Stromdichte erzeugt.

Aufgabe der Erfindung ist es, eine Vorrichtung der eingangs beschriebenen Art zur schaffen, die so betrieben werden kann, daß eine kontinuierliche Entkeimung ohne Unterbrechung wegen des Reinigens der Elektroden möglich ist.

Diese Aufgabe wird durch eine Vorrichtung der eingangs beschriebenen Art gelöst, die gemäß der Erfindung dadurch gekennzeichnet ist, daß in Strömungsrichtung eine Mehrzahl Hauptelektroden mit dazwischenliegenden Hilfselektroden vorgesehen sind, wobei jeweils zwei aufeinanderfolgende Hauptelektroden spannungsmäßig entgegengesetzt gepolt sind und das periodische Umschalten der Potentialrichtung in einer Periode jeweils nur zwischen zwei benachbarten Hauptelektroden erfolgt.

Bei einer besonderen Ausführungsform der Erfindung erfolgt die Umschaltung der Potentialrichtung zyklisch jeweils nacheinander an den Paaren n, n+1; n+2, n+3; ...; n+i, n+i+1 (n=1,2,3,... und i=gerade Zahl). So wird ermöglicht, daß nur der zwischen zwei benachbarten Hauptelektroden liegende Bereich für der Entkeimung ausffällt.

Die die Hauptelektroden miteinander und mit der Spannungsquelle verbindenden Kontaktmittel sind gemäß der Erfindung außerhalb des Strömungsbereiches angeordnet, so daß die Gleichmäßigkeit der Strömung bzw. Durchwirbelung nicht beeinflußt wird.

Nach einer Ausführungsform der Erfindung sind die Hauptelektroden durch eine Mehrzahl von in einer Ebene quer zur Strömungsrichtung liegenden elektrisch miteinander verbundenen Elektrodenstäben, die zueinander parallel und in einem Abstand voneinander angeordnet sind, und die Nebenelektroden durch in dazu parallelen Ebenen elektrisch gegeneinander isoliert angeordnete Stäbe, die jeweils zu den Stäben der benachbarten Schicht versetzt angeordnet sind, gebildet. Durch diese geometrische Ausbildung wird eine starke Durchwirbelung und Abscherung sich auf den Elektroden bildender Bläschen erreicht, wodurch der Wirkungsgrad verbessert wird.

Weitere Merkmale der Erfindung sind in den Unteransprüchen gekennzeichnet.

Die durch die Erfindung erreichten Vorteile bestehen insbesondere in der Schaffung einer Vorrichtung zur anodischen Oxidation, mit der ein kontinuierliches Entkeimen mit hohem Wirkungsgrad erreicht wird.

Im folgenden wird die Erfindung anhand von lediglich Ausführungswege darstellenden Zeichnungen näher erläutert. Es zeigen:

Fig. 1 eine perspektivische Darstellung einer Zelle zum Entkeimen von Flüssigkeiten, teilweise in gebrochener Darstellung;

Fig. 2 eine abgewandelte Ausführungsform der Hauptund Hilfselektroden in perspektivischer Darstellung;

Fig. 3 eine Draufsicht auf eine andere Ausführungsform von in einer Ebene liegenden Elektroden;

Fig. 4 eine perspektivische Seitenansicht einer weiteren Ausführungsform von Elektoden; und

Fig. 5 eine Seitenansicht einer Ausführungsform mit Steuereinrichtung.

Die Vorrichtung besteht aus einem aus elektrisch isolierendem Kunststoff gebildeten Zellengehäuse 1 mit eine Eingang 2 am unteren Teil des Zellengehäuses und einem Ausgang 3 am oberen Teil zum Ableiten der entkeimten Flüssigkeit. Über dem Eingang 2 ist über den ganzen inneren Querschnitt des Zellengehäuses ein siebartiger Flüssigkeitsgleichrichter 4 vorgesehen. In dem darüber befindlichen Raum ist eine Vielzahl von Elektrodenstäben 8,9 vorgesehen, die jeweils in in der Gehäusewand vorgesehene Löcher eingeschoben und mit einem geeigneten Kitt abgedichtet gehalten werden.

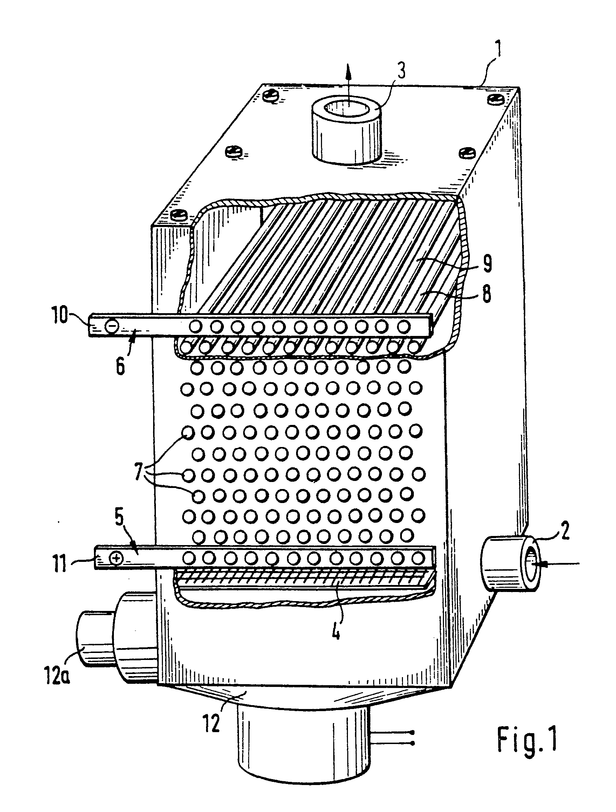

Die Elektrodenstäbe sind senkrecht zur Strömungsrichtung und parallel zueinander angeordnet, wobei jeweils eine Gruppe von Elektrodenstäben in einer Ebene liegt und die verschiedenen Ebenen solcher Elektrodenstäbe einen Abstand voneinander aufweisen. Ferner sind die Elektrodenstäbe einer Ebene jeweils in der aus der Figur 1 ersichtlichen Weise um einen halben Abstand zwischen zwei benachbarten Stäben einer Ebene seitlich gegen die Stäbe der nächt folgenden Ebene versetzt.

Die unterste Reihe Elektrodenstäbe ist mit einem Verbindungselement 11 und die oberste Reihe der Elektrodenstäbe durch ein Verbindungselement 10 elektrisch zusammengefaßt. An die Verbindungselemente 10,11 werden über Zuleitungen die entsprechenden Potentiale angelegt. Zur Vereinfachung sind in Fig. 1 nur zwei am Potential angelegte Reihen gezeigt. Im weiteren werden die mit der Spannungsquelle verbundenen Elektroden als Hauptelektroden und die dazwischenliegenden Elektroden als Hilfselektroden bezeichnet.

Im Betrieb läuft die in der Regel schwach leitende Flüssigkeit durch den Eingang 2 unten in das Zellengehäuse 1 hinein, durch den Flüssigkeitsgleichrichter hindurch und umströmt von unten nach oben die Hauptelektrode 5, die zwischen den beiden Haupteletroden 5 und 6 liegenden Nebenelektroden 7 und die zweite Hauptelektrode 6 und tritt durch den Ausgang 3 nach außen.

Die einzelnen Gitterelektroden sind im definierten Längsteilungsverhältnis $S_1/d$ zu einem vertikal oder horizontal durchströmten Bündel zusammengefaßt. Dabei können die Stab- oder Drahtlagen von Elektrode zu Elektrode als versetztes, fluchtendes oder unregelmäßiges Parallel- oder Kreuzgitterbündel angeordnet sein. Für die weitere Beschreibung wird vom Parallelgitterbündel mit versetzten Stäben und vertikal von unten nach oben durchströmten Elektroden ausgegangen. Wird das Parallelgitterbündel mit versetzten Stäben durchströmt, so wirken aufgrund der starken Querschnittsänderungen und Umlenkungen hohe Beschleunigungs- und Verzögerungskräfte längs der Strombahn auf das Fluid. Diese bewirken wiederum eine intensive Wirbelbewegung zwischen den einzelnen Elektrodenlagen. Die Intensität der Wirbelbewegung und somit der Durchmischung im Fluid läßt sich bei konstanter Reynoldszahl $R_e$ durch Widerstandszahl $\psi$ und den Druckverlust $\Delta P$ ausdrücken. Dabei ist nur noch eine Abhängigkeit von dem Querverteilungsverhältnis $Sq/d$, nicht aber vom Längsteilungsverhältnis $S_1/d$ gegeben, da der Strömungswiderstand vornehmlich durch die Umlenkungen bestimmt wird:

$$\psi - Re^{-0,16} \quad [1+\frac{0,47}{(Sq/d)^{1,08}}]$$

$$\psi = \frac{\Delta P/n_1}{\Sigma \, w^2/2}$$

$$Re = \frac{w \cdot d}{\nu}$$

Es bedeuten $\Delta P$ Druckverlust, w Geschwindigkeit im engsten freien Querschnitt, d Stabdurchmesser, $\Sigma$ und $\nu$ Dichte und Kinematische Viskosität des Fluids und $n_1$ die Zahl der in Strömungsrichtung hintereinander liegenden Stablagen. Die oben genannten Gleichungen gelten nur für $n_1 > 10$, andernfalls erhölt sich die Widerstandszahl.

Aufgrund der hohen Strömungsgeschwindigkeit in den engsten freien Querschnitten des Stabgitterbündels und den ausgeprägten Wirbelgebieten hinter jedem einzelnen Stab werden die Adhäsionskräfte der Gasblasen auf den Staboberflächen schon im Anfangsstadium ihrer Entstehung durch Schwerkräfte des Fluids überwunden, so daß für eine stetige Gasabfuhr und somit für eine ständige Aktivierung der Elektrodenoberfläche gesorgt ist. Damit an den Gasblasen, welche die Staboberfläche in einer sehr dünnen Schicht bedecken, Scherkräfte wirksam werden können, ist eine geringe Grenzschichtdicke Voraussetzung. Diese ist bei dünnen Stäben (d=1 bis 3mm) aufgrund des kurzen Strömungsweges senkrecht zur Stabachse gegeben. Das die Elektroden bildende Mittel, also die Stäbe bzw. das Geflechtmaterial hat daher vorzugsweise einen Durchmesser von 1 bis 10 und ganz besonders 1 bis 4 mm und der Abstand der Elektroden voneinander ist so gewählt, daß die Strömungsschichtdicke etwa die Hälfte des Elektrodendurchmessers beträgt.

Die intensive Wirbelbewegung im Stabbündel bedingt ferner einen guten Fluidaustausch ander Elektrodengrenzschicht, so daß bei genügend großer Elektrodenzahl $n_1$ ein Großteil des zu entkeimenden Fluids in den Grenzschichtbereich der Stabelektroden gerät.

Bei der beschriebenen Anordnung mit durchströmten Elektroden werden die wässrige Lösung und die in ihr enthaltenen Keime zwangsweise durch die anodische oder kathodische Grenzschicht hindurchgeführt. Dabei wird das gesamte Potentialgefälle von 0 bis beispielsweise 11 V durchwandert, so daß im Vergleich zu tangential angeströmten Elektroden, gleichgültig welcher Art, durch beide Effekte eine höhere chemische Umsetzungsrate erzielt wird.

Grundsätzlich können nich nur die beiden die Hauptelektroden 5, 6 bildenden Reihen von Stabelektroden an entsprechende Potentiale sondern auch jede Reihe von Stabelektroden einzeln angeschlossen werden. Eine solche mehrfach monopolare Schaltung hat den Vorteil, daß die Gesamtspannung auf eine Bruchteil herabgesetzt werden kann. Wird beispielsweise Leitungswasser behandelt, so sind dazu wegen der geringen Leitfähigkeit ca. 600 V erforderlich, die bei einer Aufteilung in drei bipolare Bereiche auf jeweils 200 V herabgesetzt werden kann. Bei entsprechender Unterteilung ist es möglich, bei hohen Strömen schon mit sehr geringen Spannungen in der Größenordnung von 2 bis 3 V auszukommen, was ermöglicht, eine derartige Vorrichtung als Entkeimungsanlage in sehr sonnenreichen Ländern über Solarzellen, die nur geringe Spannungen erzeugen, ohne Transformation zu betreiben.

Eine weitere Steigerung der Gasblasenabfuhr läßt sich durch Vibration der Elektroden im niederfrequenten Bereich erzielen. Zu diesem Zweck ist am Boden des Zellengehäuses ein schematisch dargestellter schwinger 12a zur Erzeugung niederfrequenter Schwingungen vorgesehen. Die von diesem erzeugten Wellen werden über die Gehäuse-wandung auf die Elektrodenstäbe übertragen. Durch die Vibration werden die Adhäsionskräfte der Gasblasen auf der schwingenden Elektrodenoberfläche wesentlich vermindert, so daß die Abfuhr durch den Flüssigkeitsstrom weiter erleichtert wird.

Anstelle des oben beschriebenen periodischen Umpolens der Elektroden kann die trotz der guten Wirbelbildung sich auf der Kathodenseite bildende Karbonatablagerung, die einen Teil der Elektrodenoberfläche inaktivieren würde, durch die von einer Ultraschallquelle 12 erzeugte Vibration beseitigt werden.

Im Laufe des Betriebes kann ein Elektrodenstab durch Beschichtung aufgrund der dauernden Benutzung zerstört werden. Durch die beschriebene Befestigungsweise der Elektrodenstäbe in der Wandung des Zellengehäuses kann jeder einzelne Elektrodenstab durch einfaches Herausdrücken auf die eine Seite und anschließendes Herausziehen des Stabes entfernt und durch einfaches Einschieben und anschließendes Verkitten eines neuen Stabes ausgewechselt werden.

Ergänzend sei ausgeführt, daß der Abstand der Stäbe einen direkten Einfluß auf die Wirtschaftlichkeit der Zelle hat, weil das Fluid mit dem ihm zukommenden Ohm'schen Widerstand einen elektrischen Verlust zufolge hat. Der elektrische Verlust läßt sich dadurch reduzieren, daß der Abstand so klein wie möglich gemacht wird. Die untere Grenze für den Abstand zwischen den Stäben wird von dem zu behandelnden Medium und den darin befindlichen Verunreinigungen bestimmt. Der Abstand der Stäbe muß nämlich mindestens so groß sein, daß von der Flüssigkeit mitgeführte Partikelchen nicht zwischen den Elektrodenstäben hängenbleiben, da es sonst zu einer Verstopfung kommt. Der Abstand der Elektrodenstäbe wird also so gewählt, daß er gerade etwas größer ist als der maximale Durchmesser der von der zu behandelnden Flüssigkeit mitgeführten Teilchen.

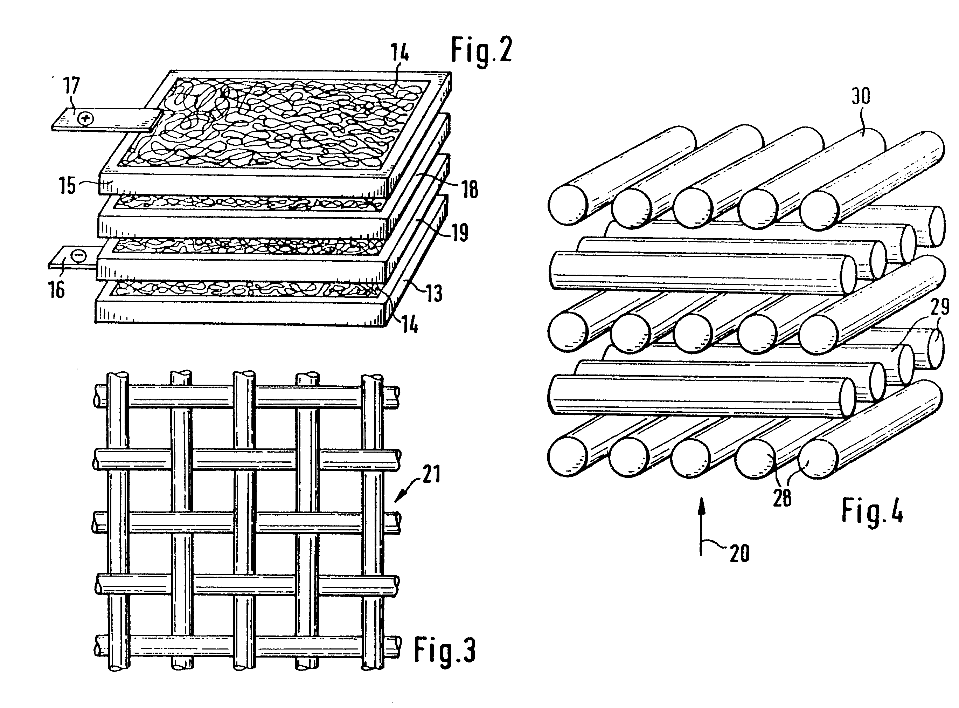

In Fig. 2 ist eine andere Ausführungsform der Hauptund Hilfselektroden gezeigt. Jede Elektrode besteht aus einem Rahmen 13 aus einem leitenden Metall und einem mit diesem elektrisch verbundenen und von dem Rahmen gehaltenen Drahtpreßgewebe 14. Die Rahmen wersen nach Abnehmen eines Deckels einzeln in das Zellengehäuse eingesetzt und durch isolierende Abstandshalter in einem Abstand voneinander gehalten. Die jeweils äußersten Rahmen 13, 15 sind mit elektrischen Anschlußelementen 16, 17 verbunden, an die das jeweilige elektrische Potential angelegt wird. Die Zwischenrahmen 18, 19, die die Nebenelektroden bilden, sind in dem gezeigten Beispiel nicht direkt mit Spannungsquellen verbunden.

In Figur 3 ist in vergößertem Maßstab ein Metallgeflecht 21 gezeigt, welches als eine Elektrodenebene verwendet werden kann. Bei dieser Ausführungsform werden mehrere solche geflochtenen Ebenen in einem Abstand voneinander und isoliert gegeneinander in das Zellengehäuse eingesetzt, wobei wie bei dem in Figur 2 gezeigten Ausführungsbeispiel an die beiden äußersten Ebenen, die die Hauptelektroden bilden, das Potential angelegt wird, während die dazwischen liegenden Ebenen die Nebenelektroden bilden. In Figur 4 ist in vergrößertem Maßstab eine Elektrodenanordnung gezeigt, bei der die Elektrodenstäbe 28, 29, 30 jeweils in einer Ebene parallel zueinander und in jeweils aufeinanderfolgenden Ebenen unter einem rechten Winkel zueinander angeordnet sind, so daß eine Art Kreuzgitterbündel entsteht. Die Elektrodenstäbe sind wie bei dem in Figur 1 gezeigten Ausführungsbeispiel in der Gehäusewandung befestigt und gegeneinander isoliert. Lediglich die Stäbe 28, 30 der beiden äußersten Ebenen sind wiederum durch Verbindungselemente elektrische miteinander verbunden, so daß das Potential an diese als Hauptelektroden wirkenden Elektrodenebenen angelegt werden kann. Die Durchströmung dieses Kreuzgitterbündels erfolgt in der durch den Pfeil 20 angedeuteten Richtung senkrecht zu den Ebenen und erfindungsgemäß annähernd senkrecht von unten nach oben, damit die abgeschiedenen Bläschen leicht aus dem Anodenbereich austreten können.

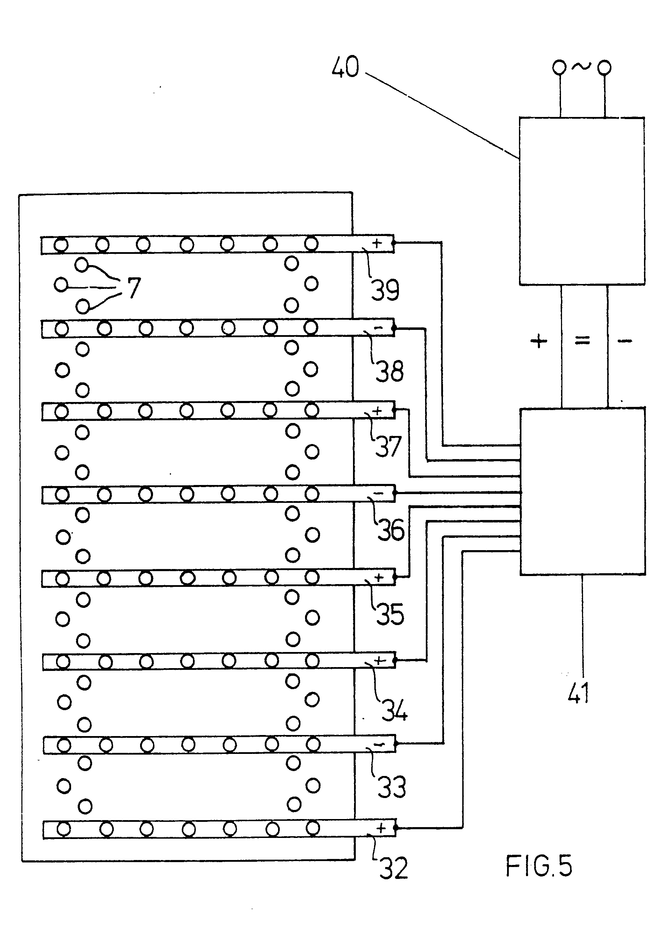

Bei der in Figur 5 gezeigten Ausführungsform sind senkrecht übereinander eine Mehrzahl von Hauptelektroden 32 bis 39 mit jeweils zwischen zwei benachbarten Hauptelektroden einer Mehrzahl von gegeneinander versetzt angeordneter Schichten von Nebenelektroden vorgesehen. In der Figur sind für jede Schicht der Nebenelektroden zur Vereinfachung der Zeichnung nur einige angedeutet.

Damit die Vorrichtung im kontinuierlichen Betrieb ohne Unterbrechung bei Umpolung zu Reinigungszwecken arbeiten kann, erfolgt die Umpolung der an der Spannungsversorgung 40 anzulegenden Spannung mittels einer Steuerung 41 jeweils nur zwischen zwei benachbarten Hauptelektroden, und zwar paarweise zwischen der ersten und zweiten Elektrode 32, 33, dann, wie in Figur 5 dargestellt, zwischen der dritten und vierten Elektrode 34, 35, dann zwischen der fünften und sechsten Hauptelektrode 36, 37 und schließlich zwischen der siebenten und achten Hauptelektrode 38, 39 oder allgemeiner ausgedrückt, zwischen der n-ten und der n+1-ten, dann zwischen der n+2-ten und n+3-ten und so fort. Auf diese Weise wird erreicht, daß bei Umschalten des Paares, n, n+1 lediglich der Bereich zwischen den Hauptelektroden n+1 und n+2, in dem in Fig. 5 gezeigten Schaltbeispiel also der Bereich zwischen den Hauptelektroden 34, 35 für die Entkeimung ausfällt, alle anderen Bereiche aber kontinuierlich arbeiten.

## Patentansprüche

1. Vorrichtung zur Entkeimung von Flüssigkeiten mittels der anodischen Oxidation mit einem Flüssigkeitseingang (2) im unteren Innenraumbereich und einem Ausgang (3) im oberen Innenraumbereich und einer Mehrzahl von durchströmten bzw. umströmten Haupt- und Hilfselektroden (32—39; 7), wobei die Hauptelektroden am stromabwärtsgelegenen Eingang und am stromaufwärtsgelegenen Ausgang des Elektrodenbereiches sich über den ganzen Strömungsquerschnitt erstrecken und Unterbrechungen zum Durchtritt der Flüssigkeit aufweisen und mit einem an den Hauptelektroden anliegenden in seiner Richtung periodisch umschalbaren Potentail, dadurch gekennzeichnet, daß in Strömungsrichtung eine Mehrzahl Hauptelektroden (32—39) mit dazwischenliegenden Hilfselektroden vorgesehen sind, wobei jeweils zwei aufeinanderfolgende Hauptelektroden (33, 34) spannungsmäßig entgegengesetzt gepolt sind und das periodische Umschalten der Potentialrichtung in einer Periode jeweils nur zwischen zwei benachbarten Hauptelektroden (34, 35) erfolgt.

2. Vorrichtung nach Anspruch 1, dadurch gekennzeichnet, daß die Umschaltung der Potentialrichtung jeweils nacheinander paarweise an der n-ten und (n+1)-ten; (n+2)-ten und (n+3)-ten,... Hauptelektrode erfolgt.

3. Vorrichtung nach Anspruch 1 oder 2, dadurch gekennzeichnet, daß die die Hauptelektroden (5, 6) miteinander und mit der Spannungsquelle verbindenden Kontaktmittel (10, 11) außerhalb des Strömungsbereiches liegen.

4. Vorrichtung nach einem der Ansprüche 1 bis 3, dadurch gekennzeichnet, daß die Hauptelektroden (5, 6) durch eine Mehrzahl von in einer Ebene quer zur Strömungsrichtung liegenden elektrisch miteinander verbundenen Elektrodenstäben (8, 9), die zueinander parallel und in einem Abstand voneinander angeordnet sind, und die Nebenelektroden durch in dazu parallelen Ebenen elektrisch gegeneinander isoliert angeordnete Stäbe (7), die jeweils zu den Stäben der benachbarten Schicht versetzt angeordnet sind, gebildet sind.

5. Vorrichtung nach einem der Ansprüche 1 bis 3, dadurch gekennzeichnet, daß die Elektroden als in Strömungsrichtung hintereinanderliegende Drahtpreßgewebe (14) ausgebildet sind.

6. Vorrichtung nach einem der Ansprüche 1 bis 5, dadurch gekennzeichnet, daß die die Elektroden bildenden Mittel einen Durchmesser von etwa 1 bis 10 mm und vorzugsweise 1 bis 4 mm haben und ihr Abstand so gewählt ist, daß die Strömungsschichtdicke etwa die Hälfte des Elektrodendurchmessers beträgt.

7. Vorrichtung nach einem dur Ansprüche 1 bis 6, dadurch gekennzeichnet, daß an dem Gehäuse (1) eine auf die Elektroden Schwingungen übertragende Ultraschallquelle (12) vorgesehen ist.

8. Vorrichtung nach einem der vorhergehenden Ansprüche, dadurch gekennzeichnet, daß an dem Gehäuse (1) ein auf die Elektroden Schwingungen übertragender Schwinger (12a) zur Erzeugung niederfrequenter Schwingungen vorgesehen ist.

## Claims

1. An apparatus for disinfection of liquids by the anodic oxidation with an input of liquid (2) in the lower inside-room-area and an outflow (3) in the upper inside room-area and a plurality of main- or auxiliary electrodes (32—39;7), which are streamed through or circulated around. In this apparatus the main-electrodes at the downstream lying inflow and at the upstream lying outflow of the electrode-area extend over the whole streaming-profile and shows interruptions passing through the fluid medium. In this apparatus a potential lies at the main-electrodes, which is periodical commutatible in this direction. This apparatus is characterized in, that a plurality of main-electrodes (32—39) and between lying auxiliary electrodes, which are arranged in layers in the direction of the streaming. Consecutive main-electrodes have a inverse voltage polarity and the periodical commutation of the potential-direction comes in one period to pass only between two neighboured main-electrodes (34,35).

2. The apparatus according to claim 1, characterized in, that the commutation of the potential-direction occurs in pairs at the $n^{th}$ and the $(n+1)^{th}$; $(n+2)^{th}$ and $(n+3)^{th}$,... main-electrode.

3. The apparatus according to claim 1 and 2, characterized in, that the catalysts (10,11), which connect the main-electrodes (5,6) together and with the voltage-source, lie out of the streaming-zone.

4. The apparatus according to the claims 1 to 3, characterized in, that the main-electrodes (5,6) are disposed in a layer traverse to the streaming direction with a plurality of electrode-bars (8,9) together electrical contacted, which lie parallel in a defined distance, characterized in, that the auxiliary electrodes are composed in parallel layers as electrical head-on isolated electrode bars (7), which are shifted to the electrode bars of the neighboured electrode-layer.

5. The apparatus according to the claims 1 to 3, characterized in, that the electrodes are formed as wire-press-texture, which lies in series in the streaming direction (14).

6. The apparatus according to the claims 1 to 5, characterized in, that the medium, which forms the electrodes approximately has a diameter of 1 to 10 mm and preferred 1 to 4 mm and a distance between them of approximately half of the electrode-bar diameter.

7. The apparatus according to the claims 1 to 6, characterized in, that at the case (1) a ultrasonic-Source (12) transmit oscillations to the electrodes.

8. The apparatus according to one of the previous claims, characterized in, that at the case (1) an oscillator (12a) exists, which generates and transmits low frequency oscillations to the electrodes.

## Revendications

1. Appareil pour la stérilisation des fluides au moyen de l'oxydation anodique, doté d'une entrée de fluide (2) dans le domaine intérieur inférieur et d'une sortie (3) dans le domaine intérieur supérieur et de plusieurs électrodes principales et secondaires, les premières étant passantes au fluide et les secondes étant contournées par le fluide (32—39; 7), où les électrodes principales, stiuées en aval de l'entrée et en amont de la sortie du domaine des électrodes couvrent la totalité de la section de l'écoulement et présentent des barrages au passage du fluide, doté d'un potentiel, s'inversant périodiquement de sens, appliqué aux électrodes principales; cet apparail qui est caractérisé par le fait que, dans la direction de l'écoulement, plusieurs électrodes principales (32—39) avec des électrodes secondaires intermédiaires sont prévues (33, 34), où chaque fois deux électrodes principales consécutives sont portées à une tension égale et de signe opposé et où le changement périodique du sens du potentiel dans une période n'a lieu respectivement qu'entre deux électrodes voisines (34, 35).

2. Appareil d'après le titre 1, caractérisé par le fait que le changement du sens du potentiel a lieu chaque fois successivement et sur une paire, sur la n-ième et (n+1)-ième, (n+2)-ième et (n+3)-ième, ... électrode principale.

3. Appareil d'après le titre 1 ou 2, caractérisé par le fait que les moyens de contact (10, 11) qui relient les électrodes principales (5, 6) entre elles et à la source de tension sont situés à l'extérieur de domaine de l'écoulement.

4. Appareil d'après l'un des titres 1 à 3, caractérisé par le fait que, les électrodes principales (5, 6) sont constituées par plusieurs tiges électrodes (8, 9), reliées électriquement entre elles, qui sont situées sur un plan perpendiculaire à la direction de l'écoulement, qui sont parallèles entre elles et disposées à une certaine distance les unes des autres; et que les électrodes secondaires sont constituées par des tiges (7) isolées électriquement entre elles, disposées sur plan parallèle au précédent, et qui sont placées dans les interstices da la couche voisine.

5. Appareil d'après les titres 1 à 3, caractérisé par le fait que les électrodes sont construites en tant que grilles de fils pressés (14) disposées les unes derrière les autres dans la direction de l'écoulement.

6. Appareil d'après les titres 1 à 5, caractérisé par le fait que la matière qui constitue les tiges électrodes a un diamètre de environ 1 à 10 mm, de préférence 1 à 4 mm, et que leur écartement est choisi de telle facon que l'épaisseur de la couche d'écoulement soit environ la moitié du diamètre de l'électrode.

7. Appareil d'après les titres 1 à 6, caractérisé par le fait qu'une source d'ultrasons (12) qui transmet les oscillations aux électrodes est prévue au boitier (1).

8. Appareil d'après l'un des titres précédents, caractérisé par le fait qu'un oscillateur (12a), destiné à la génération d'oscillations basse fréquence, et qui transmet les oscillations aux électrodes, est prévu au boitier (1).

Fig.1

Fig.2

14

17 ⊕

15

16 ⊖

18

19

13

14

Fig.3

21

30

29

28

20

Fig.4

2

0 001 285

FIG.5